# FASCICULE DE BREVET EUROPEEN

(11) **EP 1 068 856 B1**
(45) Date de publication et mention de la délivrance du brevet: **02.04.2003**
(21) Numéro de dépôt: 00401991.5
(22) Date de dépôt: 10.07.2000
(51) Int. Cl.: A61K 7/027, A61K 7/032, C07D 209/08

(54) **Composition cosmétique sans cire, structurée sous forme rigide par un polymère**
Wachsfreie strukturierte kosmetische Zusammensetzung, verfestigt mittels eines Polymers
Wax-free structured cosmetic composition made rigid by a polymer

(30) Priorité: 15.07.1999 FR 9909177; 24.01.2000 FR 0000921
(43) Date de publication de la demande: 17.01.2001
(73) Titulaire: L'OREAL, 75008 Paris (FR)
(72) Inventeur: Ferrari, Véronique, 94700 Maison-Alfort (FR); Simon, Pascal, 94400 Vitry sur Seine (FR)
(74) Mandataire: Brédeville, Odile Marie

(56) Documents cités:
- EP-A- 0 602 905
- WO-A-95/11000
- WO-A-97/25960
- WO-A-98/17705
- US-A- 5 437 859

## Description

La présente invention se rapporte à une composition de soin et/ou de traitement et/ou de maquillage de la peau, y compris du cuir chevelu, et/ou des lèvres des êtres humains, contenant une phase grasse liquide, gélifiée par un polymère particulier se présentant notamment sous forme d'un stick de maquillage comme les rouges à lèvres, dont l'application conduit à un dépôt brillant et non-migrant.

Dans les produits cosmétiques ou dermatologiques, il est courant de trouver une phase grasse liquide structurée, à savoir gélifiée et/ou rigidifiée ; ceci est notamment le cas dans les compositions solides comme les déodorants, les baumes et les rouges à lèvres, les produits anti-cerne et les fonds de teint coulés. Cette structuration est obtenue à l'aide de cires ou de charges. Malheureusement, ces cires et charges ont tendance à matifier la composition, ce qui n'est pas toujours souhaitable en particulier pour un rouge à lèvres ; en effet, les femmes sont toujours à la recherche d'un rouge à lèvres sous forme d'un bâton déposant un film de plus en plus brillant.

Par phase grasse liquide, au sens de la demande, on entend une phase grasse liquide à température ambiante (25°C), composée d'un ou plusieurs corps gras liquides à température ambiante, appelés aussi huiles, compatibles entre eux.

La structuration de la phase grasse liquide permet en particulier de limiter son exsudation des compositions solides et, en plus, de limiter, après dépôt sur la peau ou les lèvres, la migration de cette phase dans les rides et ridules, ce qui est particulièrement recherché pour un rouge à lèvres. En effet, une migration importante de la phase grasse liquide, chargée de matières colorantes, conduit à un effet inesthétique autour des lèvres, accentuant particulièrement les rides et les ridules. Cette migration est souvent citée par les femmes comme un défaut majeur des rouges à lèvres classiques.

La brillance est liée pour l'essentiel à la nature de la phase grasse liquide. Ainsi, il est possible de diminuer le taux de cires et de charges de la composition pour augmenter la brillance d'un rouge à lèvres mais alors, la migration de la phase grasse liquide augmente. Autrement dit, les taux de cires et de charges nécessaires à la réalisation d'un stick de dureté convenable sont un frein à la brillance du dépôt.

Le demandeur a trouvé que la perte de brillance d'un stick contenant des cires était liée à la structure cristalline anisotrope de ces composés. Il a donc envisagé la fabrication d'un stick, sans cire.

L'invention a justement pour objet une composition de soin et/ou de maquillage et/ou de traitement de la peau et/ou des lèvres du visage permettant de remédier à ces inconvénients.

De façon surprenante, le demandeur a trouvé que l'utilisation de polymères particuliers permettait de structurer, même en l'absence de cire, les phases grasses liquides sous forme de stick dont l'application sur les lèvres conduisait à un film brillant et non migrant.

Le document WO 98/117705 décrit des compositions cosmétiques contenant une résine de polyamide. Cependant, ce document ne mentionne pas que ces compostions sont solides, présentent une certaine dureté et que la structuration de ces compositions peut être obtenue sans cire. Ce document n'enseigne pas un stick brillant solide, sans cire, contenant cette résine polyamide.

L'invention s'applique non seulement aux produits de maquillage des lèvres comme les rouges à lèvres, les crayons à lèvres, mais aussi aux produits de soin et/ou de traitement de la peau, y compris du cuir chevelu, et des lèvres, comme les produits en stick de protection solaire de la peau du visage ou des lèvres, aux produits de maquillage de la peau, aussi bien du visage que du corps humain, comme les fonds de teints coulés en stick ou en coupelle, les produits anti-cerne et les produits de tatouage éphémère, aux produits d'hygiène corporelle comme les déodorants en stick, et aux produits de maquillage des yeux comme les eye-liners sous forme de crayon et les mascaras pains.

L'invention a pour objet une composition structurée contenant au moins une matière colorante et une phase grasse continue liquide, structurée par au moins un polymère de masse moléculaire moyenne en poids allant de 1000 à 30 000 et mieux de 1 000 à 10 000, comportant a) un squelette polymérique, ayant des motifs de répétition hydrocarbonés pourvus d'au moins un hétéroatome, et b) au moins une chaîne grasse pendante et/ou au moins une chaîne grasse terminale, éventuellement fonctionnalisées, ayant de 12 à 120 atomes de carbone, liées à ces motifs, ces chaînes grasses représentant de 40 à 98 % du nombre total des motifs à hétéroatome et des chaînes grasses, ladite composition se présentant sous forme d'un solide exempt de cire, la matière colorante, la phase grasse liquide et le polymère formant un milieu physiologiquement acceptable.

De façon plus précise, l'invention se rapporte à une composition selon la revendication 1.

"Par au moins une chaîne grasse", on entend une ou plusieurs chaînes grasses.

Une cire, au sens de la présente invention, est un composé gras lipophile, solide à température ambiante (25°C), à changement d'état solide/liquide réversible, ayant une température de fusion supérieure à 40°C pouvant aller jusqu'à 200° C, et présentant à l'état solide une organisation cristalline anisotrope. La taille des cristaux est telle que les cristaux diffractent et/ou diffusent la lumière, conférant à la composition un aspect trouble, plus ou moins opaque. En portant la cire à sa température de fusion, il est possible de la rendre miscible aux huiles et de former un mélange homogène microscopiquement, mais en ramenant la température du mélange à la température ambiante, on obtient une recristallisation de la cire dans les huiles du mélange. C'est cette recristallisation dans le mélange qui est responsable de la diminution de la brillance dudit mélange.

Les cires, au sens de la demande, sont celles généralement utilisées dans les domaines cosmétique et dermatologique; elles sont notamment d'origine naturelle comme la cire d'abeilles, la cire de Carnauba, de Candellila, d'Ouricoury, du Japon, de fibres de liège ou de canne à sucre, les cires de paraffine, de lignite, les cires microcristallines, la cire de lanoline, la cire de Montan, les ozokérites, les huiles hydrogénées comme l'huile de jojoba hydrogénée, mais aussi d'origine synthétique comme les cires de polyéthylène issues de la polymérisation de l'éthylène, les cires obtenues par synthèse de Fischer-Tropsch, les esters d'acides gras et les glycérides concrets à 40°C, les cires de silicone comme les alkyle, alcoxy et/ou esters de poly(di)méthylsiloxane solide à 40°C.

La composition de l'invention est avantageusement autoportée et peut se présenter sous forme de stick ou de coupelle. Elle se présente en particulier sous forme d'un gel rigide anhydre transparent, et plus spécialement sous forme de stick anhydre transparent, la phase grasse liquide formant la phase continue.

La structure ou gélification de la phase grasse liquide (ou huile), qui est modulable par la nature du polymère à hétéroatome utilisé, est telle que l'on obtienne une structure rigide sous forme d'un bâton ou d'un stick. Ces bâtons lorsqu'ils sont colorés permettent, après application, d'obtenir un dépôt brillant, homogène en couleur et ne migrant pas dans les rides et ridules de la peau, entourant en particulier les lèvres, mais aussi les yeux. Les polymères, objets de l'invention sont solubles dans une grande diversité d'huiles.

L'invention a aussi pour objet une composition structurée telle que définie dans la revendication 3.

L'invention a encore pour objet une composition structurée de maquillage de la peau, des lèvres et/ou des phanères, contenant au moins un pigment en quantité suffisante pour maquiller la peau, les lèvres et/ou les phanères et une phase grasse continue liquide, structurée par au moins un polymère de masse moléculaire moyenne en poids allant de 1 000 à 30 000, comportant a) un squelette polymérique, ayant des motifs de répétition hydrocarbonés pourvus d'au moins un hétéroatome, et b) au moins une chaîne grasse pendante et/ou au moins une chaîne grasse terminale ayant de 12 à 120 atomes de carbone, liées à ces motifs, les chaînes grasses représentant de 40 à 98 % du nombre total des motifs à hétéroatome et des chaînes grasses, ladite composition se présentant sous forme d'un solide, le pigment, la phase grasse liquide et le polymère formant un milieu physiologiquement acceptable, la composition comprenant, en outre, au moins un composé amphiphile liquide à température ambiante, de valeur de HLB inférieur à 12.

En particulier, cette composition est une composition de rouge à lèvres ou un stick de maquillage de la peau, des lèvres et/ou des phanères, et en particulier des lèvres.

"Par pigment", il faut comprendre toute particule solide insoluble dans le milieu servant à donner et/ou modifier une couleur et/ou un aspect Irisé.

De façon avantageuse, le polymère de la composition de l'invention comprend une masse moléculaire moyenne en poids allant de 1 000 à 10 000 et mieux de 2000 à 8000.

Le polymère structurant de la composition de l'invention est un solide non déformable à température ambiante (25°C) et pression atmosphérique (760 mm de Hg). Il est capable de structurer la composition sans l'opacifier.

Par "chaîne fonctionnalisée" au sens de l'invention, on entend une chaîne alkyle comportant un ou plusieurs groupes fonctionnels ou réactifs notamment choisis parmi les groupes hydroxyle, éther, oxyalkylène ou polyoxyalkylène, acide carboxylique, amine, halogène, dont les groupes fluorés ou perfluorés, ester, siloxane, polysiloxane. En outre, les atomes d'hydrogène d'une ou plusieurs chaînes grasses peuvent être substitués au moins partiellement par des atomes de fluor.

Par "polymère", on entend au sens de l'invention un composé ayant au moins 2 motifs de répétition.

Par "motifs de répétitions" hydrocarbonés, on entend au sens de l'invention un motif ou chaînon comportant de 2 à 80 atomes de carbone, et de préférence de 2 à 60 atomes de carbone, portant des atomes d'hydrogène et éventuellement des atomes d'oxygène, qui peut être linéaire, ramifié ou cyclique, saturé ou insaturé. Ces motifs ou chaînons comprennent, en outre, chacun de un à plusieurs hétéroatomes non pendants et se trouvant dans le squelette polymérique.

En outre, le polymère de la composition de l'invention comprend avantageusement de 40 à 98 % de chaînes grasses par rapport au nombre total des chaînons à hétéroatome et des chaînes grasses et mieux de 50 à 95 %. La nature et la proportion des chaînons à hétéroatome est fonction de la nature de la phase grasse liquide et est en particulier similaire à la nature de la phase grasse. Ainsi, plus les motifs à hétéroatome sont polaires et en proportion élevée dans le polymère, ce qui correspond à la présence de plusieurs hétéroatomes, plus le polymère a de l'affinité avec les huiles polaires. En revanche, plus les chaînons à hétéroatome sont peu polaires voire apolaires ou en proportion faible, plus le polymère a de l'affinité avec les huiles apolaires.

Les chaînons ou motifs à hétéroatome comprennent chacun de un à plusieurs hétéroatomes choisis en particulier parmi les atomes d'azote, de soufre, de phosphore et leurs associations, associés éventuellement à un ou plusieurs atome d'oxygène. Ces motifs peuvent, en outre, comprendre un groupement polaire du type carbonyle. Les chaînons ou motifs à hétéroatome sont en particulier des chaînons comportant des chaînons hydrocarbonés et des chaînons siliconés formant un squelette organopolysiloxane, des chaînons amide formant un squelette du type polyamide, des chaînons "isocianates" ou mieux carbamate et/ou urée formant un squelette polyuréthane, polyurée et/ou polyurée-uréthane. De préférence, ces chaînons sont des chaînons amide. Avantageusement, les chaînes pendantes sont liées directement à l'un au moins des hétéroatomes du squelette polymérique.

Entre les motifs hydrocarbonés, le polymère peut comprendre des motifs oxyalkylénés.

Comme polymères structurant préférés utilisables dans l'invention, on peut citer les polyamides ramifiés par des chaînes grasses pendantes et/ou terminales ayant de 12 à 120 atomes de carbone et notamment de 12 à 68 atomes de carbone, les chaînes grasses terminales étant liées au squelette polyamide par des fonctions ester.

Ces polymères sont de préférence des polymères résultant d'une polycondensation entre un diacide carboxylique à au moins 32 atomes de carbone (ayant notamment de 32 à 44 atomes de carbone) avec une diamine ayant au moins 2 atomes de carbone (ayant notamment de 2 à 36 atomes de carbone). Le diacide est de préférence un dimère d'acide gras ayant au moins 16 atomes de carbone comme l'acide oléïque, linoléïque, linolénïque. La diamine est de préférence l'éthylène diamine, l'hexylène diamine, l'hexaméthylène diamine, le phénylène diamine, l'éthylène triamine et encore mieux l'éthylène diamine. Pour les polymères comportant un ou 2 groupements d'acide carboxylique terminaux, il est avantageux de les estérifier par un monoalcool ayant au moins 4 atomes de carbone, de préférence de 10 à 36 atomes de carbone et mieux de 12 à 24 et encore mieux de 16 à 24, par exemple à 18 atomes de carbone.

Ces polymères sont plus spécialement ceux décrits dans le document US-A-5783657 de la société Union Camp. Chacun de ces polymères satisfait notamment à la formule (I) suivante : 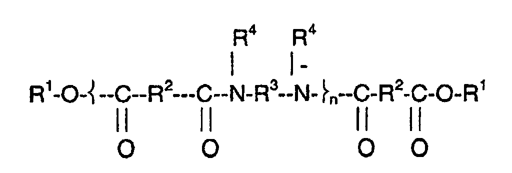 dans laquelle n désigne un nombre entier de motifs amide tel que le nombre de groupes ester représente de 10 % à 50 % du nombre total des groupes ester et amide ; R¹ est à chaque occurrence indépendamment un groupe alkyle ou alcényle ayant au moins 4 atomes de carbone et notamment de 4 à 24 atomes de carbone ; R² représente à chaque occurrence indépendamment un groupe hydrocarboné en C₄ à C₄₂ à condition que 50 % au moins des groupes R² représentent un groupe hydrocarboné en C₃₀ à C₄₂ ; R³ représente à chaque occurrence indépendamment un groupe organique pourvu d'au moins 2 atomes de carbone, d'atomes d'hydrogène et optionnellement d'un ou plusieurs atomes d'oxygène ou d'azote ; et R⁴ représente à chaque occurrence indépendamment un atome d'hydrogène, un groupe alkyle en C₁ à C₁₀ ou une liaison directe à R³ ou à un autre R⁴ de sorte que l'atome d'azote auquel sont liés à la fois R³ et R⁴ fasse partie d'une structure hétérocyclique définie par R⁴-N-R³, avec au moins 50 % des R⁴ représentant un atome d'hydrogène.

Dans le cas particulier de la formule (I), les chaînes grasses terminales éventuellement fonctionnalisées au sens de l'invention sont les chaînes terminales liées au demier hétéroatome, ici azote, du squelette polyamide.

En particulier, les groupes ester de la formule (I), qui font partie des chaînes grasses terminales et/ou pendantes au sens de l'invention, représentent de 15 à 40 % du nombre total des groupes ester et amide et mieux de 20 à 35 %. De plus, n représente avantageusement un nombre entier allant de 1 à 5. De préférence, R¹ est un groupe alkyle en C₁₂ à C₂₂ et de préférence en C₁₆ à C₂₂. Avantageusement, R² peut être un groupe hydrocarboné (alkylène) en C₁₀ à C₄₂. De préférence, 50 % au moins et mieux 75 % au moins des R² sont des groupes ayant de 30 à 42 atomes de carbone. Les autres R² sont des groupes hydrogénés en C₄ à C₁₉ et même en C₄ à C₁₂. De préférence, R³ représente un groupe hydrocarboné en C₂ à C₃₆ ou un groupe polyoxyalkyléné et R⁴ représente un atome d'hydrogène. De préférence, R³ représente un groupe hydrocarboné en C₂ à C₁₂.

Les groupes hydrocarbonés peuvent être des groupes linéaires, cycliques ou ramifiés, saturés ou insaturés. Par ailleurs, les groupes alkyle et alkylène peuvent être des groupes linéaires ou ramifiés, saturés ou non.

Selon l'invention, la structuration de la phase grasse liquide est obtenue à l'aide d'un ou plusieurs polymères de formule (I). En général, les polymères de formule (I) se présentent sous forme de mélanges de polymères, ces mélanges pouvant en outre contenir un produit de synthèse correspondant à un composé de formule (I) avec n valant 0, c'est-à-dire un diester.

A titre d'exemple de polymères structurant utilisables dans la composition selon l'invention, on peut citer les produits commerciaux vendu par la société Bush Boake Allen sous les noms Uniclear 80 et Uniclear 100. Ils sont vendus respectivement sous forme de gel à 80 % (en matière active) dans une huile minérale et à 100 % (en matière active). Ils ont un point de ramollissement de 88 à 94°C. Ces produits commerciaux sont un mélange de copolymère d'un diacide en C₃₆ condensé sur l'éthylène diamine, de masse moléculaire moyenne d'environ 6000. Les groupes ester terminaux résultent de l'estérification des terminaisons d'acide restantes par l'alcool cétylique, stéarylique ou leurs mélanges (appelés aussi alcool cétylstéarylique).

Les polymères structurant de la composition de l'invention ont avantageusement une température de ramollissement supérieure à 70°C et pouvant aller jusqu'à 190°C. De préférence, ils présentent une température de ramollissement allant de 80 à 130°C et mieux de 80°C à 105°C. Cette température de ramollissement est plus basse que celle des polymères structurant connus, ce qui facilite la mise en oeuvre des polymères objet de l'invention et limite les détériorations de la phase grasse liquide.

Les polymères objet de l'invention présentent du fait de leur chaîne grasse, une bonne solubilité dans les huiles (à savoir composés liquides, non miscibles à l'eau) et donc conduisent à des compositions macroscopiquement homogènes même avec un taux élevé (au moins 25%) de polymère, contrairement aux polymères de l'art antérieur exempts de chaîne grasse.

Avantageusement, le polymère est associé à au moins un composé amphiphile liquide à température ambiante, de valeur de balance hydrophile/lipophile (HLB) inférieure à 12 et notamment allant de 1 à 7 et de préférence de 1 à 5 et mieux de 3 à 5. Selon l'invention, on peut utiliser un ou plusieurs composés amphiphiles. Ces composés amphiphiles ont pour but de renforcer les propriétés structurantes du polymère à hétéroatome, de faciliter la mise en oeuvre du polymère et d'améliorer la capacité à déposer du stick.

Selon l'invention, la composition a de préférence une dureté allant de 0,19 à 19,6 N (20 à 2 000 gf) et de préférence de 0,19 à 8,82 N (20 à 900 gf), et mieux de 0,19 à 5,88 N (20 à 600 gf), et par exemple de 1,47 à 4,41N (150 à 450 gf). Cette dureté peut être mesurée selon une méthode de pénétration d'une sonde dans ladite composition et en particulier à l'aide d'un analyseur de texture (par exemple TA-XT2 de chez Rhéo) équipé d'un cylindre en ébonite de 25 mm de haut et 8 mm de diamètre. La mesure de dureté est effectuée à 20°C au centre de 5 échantillons de la dite composition. Le cylindre est introduit dans chaque échantillon de composition à une pré-vitesse de 2mm/s puis à une vitesse de 0,5 mm/s et enfin à une post-vitesse de 2mm/s, le déplacement total étant de 1mm. La valeur relevée de la dureté est celle du pic maximum. L'erreur de mesure est de +/- 50 gf.

La dureté peut aussi être mesurée par la méthode dite du fil à couper le beurre, qui consiste à couper un bâton de rouge à lèvres de 8,1 mm et à mesurer la dureté à 20°C, au moyen d'un dynamomètre DFGHS 2 de la société Indelco-Chatillon se déplaçant à une vitesse de 100mm/minute. Elle est exprimée comme la force de cisaillement (exprimée en gramme) nécessaire pour couper un stick dans ces conditions. Selon cette méthode la dureté d'une composition en stick selon l'invention va de 0,29 à 1,47 N (30 à 150 gf), de préférence de 0,29 à 1,18 N (30 à 120 gf) et par exemple de 0,29 à 0,49 N (30 à 50 gf).

La dureté de la composition selon l'invention est telle que la composition est autoportée et peut se déliter aisément pour former un dépôt satisfaisant sur la peau et les lèvres. En outre, avec cette dureté, la composition de l'invention résiste bien aux chocs.

Selon l'invention, la composition sous forme de stick a le comportement d'un solide élastique déformable et souple, conférant à l'application une douceur élastique remarquable. Les compositions en stick de l'art antérieur n'ont pas cette propriété d'élasticité et de souplesse.

Le ou les composés amphiphiles utilisables dans la composition de l'invention comprennent une partie lipophile liée à une partie polaire, la partie lipophile comportant une chaîne carbonée ayant au moins 8 atomes de carbone notamment, de 18 à 32 atomes de carbone et mieux de 18 à 28 atomes de carbone. De préférence, la partie polaire de ce ou ces composés amphiphiles est le reste d'un composé choisi parmi les alcools et les polyols ayant de 1 à 12 groupements hydroxyle, les polyoxyalkylènes comportant au moins 2 motifs oxyalkylénés et ayant de 0 à 20 motifs oxypropylénés et/ou de 0 à 20 motifs oxyéthylénés. En particulier, le composé amphiphile est un ester choisi parmi les hydroxystéarates, les oléates, les isostéarates du glycérol, du sorbitan ou du méthylglucose, ou encore les alcools gras ramifiés en C₁₂ à C₂₆ comme l'octyldodécanol et leurs mélanges. Parmi ces esters, on préfère les monoesters et les mélanges de mono- et de di-esters.

Le taux de composé amphiphile et celui du polymère à hétéroatome sont choisis selon la dureté de gel désirée et en fonction de l'application particulière envisagée. Les quantités respectives de polymère et de composé amphiphile doivent être telles qu'elles permettent l'obtention d'un stick délitable. En pratique, la quantité de polymère (en matière active) représente de 0,5 à 80 % du poids total de la composition et mieux de 5 à 40 %. La quantité de composé amphiphile représente en pratique de 0,1 % à 35 % du poids total de la composition et mieux de 2 % à 15 %.

Avantageusement, la phase grasse liquide de la composition contient plus de 40 % et mieux plus de 50 % d'huile(s) liquide(s) ayant un groupement similaire à celui des chaînons à hétéroatome. En particulier, la phase grasse liquide structurée par un squelette de type polyamide contient une quantité majoritaire, à savoir supérieure à 50 % du poids total de la phase grasse liquide, d'huile ou mélange d'huiles liquides apolaires notamment hydrocarbonées.

Pour une phase grasse liquide structurée par un polymère à squelette partiellement siliconé, cette phase grasse contient, de préférence, plus de 40 % et mieux plus de 50 % en poids, d'huile ou mélange d'huiles liquides siliconées, par rapport au poids total de la phase grasse liquide.

Pour une phase grasse liquide structurée par un polymère apolaire du type hydrocarboné, cette phase grasse contient avantageusement plus de 40 % et mieux plus de 50 % en poids, d'huile ou mélange d'huiles apolaires liquides, notamment hydrocarbonées, par rapport au poids total de la phase grasse liquide.

En particulier, les huiles polaires de l'invention sont :
- les huiles végétales hydrocarbonées à forte teneur en triglycérides constitués d'esters d'acides gras et de glycérol dont les acides gras peuvent avoir des longueurs de chaînes variées, ces dernières pouvant être linéaires ou ramifiées, saturées ou insaturées ; ces huiles sont notamment les huiles de germe de blé, de maïs, de tournesol, de karité, de ricin, d'amandes douces, de macadamia, d'abricot, de soja, de colza, de coton, de luzerne, de pavot, de potimarron, de sésame, de courge, d'avocat, de noisette, de pépins de raisin ou de cassis, d'onagre, de millet, d'orge, de quinoa, d'olive, de seigle, de carthame, de bancoulier, de passiflore, de rosier muscat ; ou encore les triglycérides des acides caprylique/caprique comme ceux vendus par la société Stearineries Dubois ou ceux vendus sous les dénominations Miglyol 810, 812 et 818 par la société Dynamit Nobel ;
- les huiles ou esters de synthèse de formule R₅COOR₆ dans laquelle R₅ représente le reste d'un acide gras supérieur linéaire ou ramifié comportant de 1 à 40 et mieux de 7 à 19 atomes de carbone et R₆ représente une chaîne hydrocarbonée ramifiée contenant de 1 à 40 et mieux 3 à 20 atomes de carbone, avec R₅ + R₆ ≥ 10 comme par exemple l'huile de Purcellin (octanoate de cétostéaryle), l'isononanoate d'isononyle, le benzoate d'alcool en C₁₂ à C₁₅, le myristate d'isopropyle, le palmitate d'éthyl-2-hexyle, des octanoates, décanoates ou ricinoléates d'alcools ou de polyalcools ; les esters hydroxylés comme le lactate d'isostéaryle, le malate de di-isostéaryle ; et les esters du pentaérythritol ;
- les éthers de synthèse ayant de 10 à 40 atomes de carbone;
- les alcools gras en C₈ à C₂₆ comme l'alcool oléique ;
- leurs mélanges.

Les huiles apolaires selon l'invention sont en particulier les huiles siliconées telles que les polydiméthylsiloxanes (PDMS) volatils ou non, linéaires ou cycliques, liquides à température ambiante ; les polydiméthylsiloxanes comportant des groupements alkyle, alcoxy ou phényle, pendant et/ou en bout de chaîne siliconée, groupements ayant chacun de 2 à 24 atomes de carbone; les silicones phénylées comme les phényl triméthicones, les phényl diméthicones, les phényl triméthylsiloxy diphénylsiloxanes, des diphényl diméthicones, les diphényl méthyldiphényl trisiloxanes, les 2-phényléthyl triméthylsiloxysilicates ; les hydrocarbures ou fluorocarbures linéaires ou ramifiés d'origine synthétique ou minérale, volatils ou non comme les huiles de paraffine volatiles (telles que les isoparaffines, l'isododécane) ou non volatiles et ses dérivés, la vaseline, les polydécènes, le polyisobutène hydrogéné tel que le parléam, le squalane, et leurs mélanges. De préférence, les huiles structurées, et plus spécialement celles structurées par les polyamides et en particulier ceux de formules (I) ou les polyuréthanes ou les polyurées ou les polyurée-polyuréthanes, sont des huiles apolaires du type hydrocarboné d'origine minérale ou synthétique, choisies en particulier parmi les hydrocarbures notamment les alcanes comme l'huile de parléam, les isoparaffines dont l'isododécane, le squalane et leurs mélanges.

La phase grasse liquide représente, en pratique, de 5 à 99 % du poids total de la composition, de préférence de 20 à 75 %.

La composition de l'invention peut comprendre, en outre, tout additif usuellement utilisé dans le domaine concerné, choisi notamment parmi l'eau éventuellement épaissie ou gélifiée par un épaississant ou un gélifiant de phase aqueuse, les antioxydants, les huiles essentielles, les conservateurs, des parfums, des neutralisants, des polymères liposolubles, des actifs cosmétiques ou dermatologiques comme par exemple des émollients, des hydratants, des vitamines, des acides gras essentiels, des filtres solaires, et leurs mélanges. Ces additifs peuvent être présents dans la composition à raison de 0 à 20% du poids total de la composition et mieux de 0 à 10%. Avantageusement, la composition contient au moins un actif cosmétique ou dermatologique.

Bien entendu l'homme du métier veillera à choisir les éventuels additifs complémentaires et/ou leur quantité de telle manière que les propriétés avantageuses de la composition selon l'invention ne soient pas ou substantiellement pas, altérées par l'adjonction envisagée.

La composition selon l'invention peut se présenter sous la forme d'une composition teintée dermatologique ou de soin des matières kératiniques comme la peau, les lèvres et/ou les phanères, sous forme d'une composition de protection solaire ou d'hygiène corporelle notamment sous forme de produit déodorant ou démaquillant sous forme de stick. Elle peut notamment être utilisée comme base de soin pour la peau, les phanères ou les lèvres (baumes à lèvres, protégeant les lèvres du froid et/ou du soleil et/ou du vent, crème de soin pour la peau, les ongles ou les cheveux).

La composition de l'invention peut également se présenter sous la forme d'un produit coloré de maquillage de la peau, en particulier un fond de teint, présentant éventuellement des propriétés de soin ou de traitement, un blush, un fard à joues ou à paupières, un produit anti-cerne, un eye-liner, un produit de maquillage du corps ; de maquillage des lèvres comme un rouge à lèvres, présentant éventuellement des propriétés de soin ou de traitement ; de maquillage des phanères comme les ongles, les cils en particulier sous forme d'un mascara pain, les sourcils et les cheveux notamment sous forme de crayon. En particulier, la composition de l'invention peut être un produit cosmétique contenant des actifs cosmétiques et/ou dermatologiques.

Bien entendu la composition de l'invention doit être cosmétiquement ou dermatologiquement acceptable, à savoir contenir un milieu physiologiquement acceptable non toxique et susceptible d'être appliquée sur la peau, les phanères ou les lèvres d'êtres humains. Par cosmétiquement acceptable, on entend au sens de l'invention une composition d'aspect, d'odeur et de toucher agréables.

Selon l'invention, la composition contient une matière colorante qui peut être choisie parmi les colorants lipophiles, les colorants hydrophiles, les pigments et les nacres habituellement utilisés dans les compositions cosmétiques ou dermatologiques, et leurs mélanges. Cette matière colorante est généralement présente à raison de 0,01 à 40 % du poids total de la composition, de préférence de 1 à 35 % et mieux de 5 à 25 %.

De préférence, la matière colorante contient essentiellement des pigments et/ou des nacres en vue d'obtenir un maquillage couvrant, c'est-à-dire ne laissant pas voir la peau, les lèvres ou les phanères. Les pigments permettent, en outre, de réduire le toucher collant des compositions, contrairement à des colorants solubles.

Les colorants liposolubles sont par exemple le rouge Soudan, le DC Red 17, le DC Green 6, le β-carotène, l'huile de soja, le brun Soudan, le DC Yellow 11, le DC Violet 2, le DC orange 5, le jaune quinoléine. Ils peuvent représenter de 0 à 20 % du poids de la compositions et mieux de 0,1 à 6 % (si présents).

Les pigments peuvent être blancs ou colorés, minéraux et/ou organiques, enrobés ou non. On peut citer, parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium. Les pigments peuvent représenter de 0 à 40 %, de préférence de 1 à 35 %, et mieux de 2 à 25 % du poids total de la composition.

Les pigments nacrés (ou nacres) peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth. Ils peuvent représenter de 0 à 20 % du poids total de la composition et mieux de 0,1 à 15 % (si présents).

La composition selon l'invention peut être fabriquée par les procédés connus, généralement utilisés dans le domaine cosmétique ou dermatologique. Elle peut être fabriquée par le procédé qui consiste à chauffer le polymère au moins à sa température de ramolllssement, à y ajouter le ou les composés amphiphiles, les matières colorantes et les additifs puis à mélanger le tout jusqu'à l'obtention d'une solution claire, transparente. Le mélange homogène obtenu peut alors être coulé dans un moule approprié comme un moule de rouge à lèvres ou directement dans les articles de conditionnement (boîtier ou coupelle notamment).

L'invention a encore pour objet un procédé cosmétique de soin, de maquillage ou de traitement des matières kératiniques des êtres humains et notamment de la peau, des lèvres et des phanères, comprenant l'application sur les matières kératiniques de la composition notamment cosmétique telle que définie ci-dessus.

L'invention a aussi pour objet l'utilisation d'une quantité suffisante d'au moins un polymère de masse moléculaire moyenne en poids allant de 1 000 à 30 000 et mieux de 1 000 à 10 000, comportant a) un squelette polymérique, ayant des motifs de répétition hydrocarbonés pourvus d'au moins un hétéroatome, et b) au moins une chaîne grasse pendante et/ou au moins une chaîne grasse terminale, éventuellement fonctlonnalisées, ayant de 12 à 120 atomes de carbone, liées à ces motifs, ces chaînes grasses représentant de 40 à 98 % du nombre total des motifs à hétéroatome et des chaînes grasses, dans une composition cosmétique non thérapeutique ou pour la fabrication d'une composition physiologiquement acceptable, contenant une phase grasse continue liquide et une matière colorante, pour structurer ladite composition sous forme d'un solide autoporté, exempte de cire et de dureté allant de 0,19 à 19,6 N (20 à 2 000 gf) et de préférence de 0,19 à 8,82 N (20 à 900 gf), et mieux de 0,19 à 5,88 N (20 à 600 gf), la composition comprenant en outre, au moins un composé amphiphile liquide à température ambiante, de valeur de HLB Inférieur à 12.

L'invention a encore pour objet l'utilisation d'une phase grasse continue liquide, structurée essentiellement par une quantité suffisante d'au moins un polymère de masse moléculaire moyenne en poids allant de 1 000 à 30 000 et mieux de 1 000 à 10 000, comportant a) un squelette polymérique, ayant des motifs de répartition hydrocarbonés pourvus d'au moins un hétéroatome, et b) au moins une chaîne grasse pendante et/ou au moins une chaîne grasse terminale éventuellement fonctionnallsées, ayant de 12 à 120 atomes de carbone, liées à ces motifs, ces chaînes grasses-représentant de 40 à 98 % du nombre total des motifs à hétéroatome et des chaînes grasses, dans une composition cosmétique non thérapeutique ou pour la fabrication d'une composition physiologiquement acceptable, rigide notamment autoportée et ayant par exemple une dureté allant de 0,19 à 19,6 N (20 à 2 000 gf) et de préférence de 0,19 à 8,82 N (20 à 900 gf), et par exemple de 0,19 à 5,88 N (20 à 600 gf), exempte de cire, brillante et/ou non migrante, la composition comprenant, en outre, au moins un composé amphlphile liquide à température ambiante, de valeur de HLB inférieur à 12.

L'invention a encore pour objet l'utilisation d'une phase grasse liquide continue, structurée essentiellement par une quantité suffisante d'au moins un polymère de masse moléculaire moyenne en poids allant de 1000 à 30 000, comportant a) un squelette polymérique, ayant des motifs de répétition hydrocarbonés pourvus d'au moins un hétéroatome, et b) au moins une chaîne grasse pendante et/ou au moins une chaîne grasse terminale, éventuellement fonctionnalisées, ayant de 12 à 120 atomes de carbone, liées à ces motifs, les chaînes grasses représentant de 40 à 98 % du nombre total des motifs à hétéroatome et des chaînes grasses, dans une composition cosmétique non thérapeutique ou pour la fabrication d'une composition physiologiquement acceptable, comme agent pour limiter la migration de ladite composition.

L'invention a encore pour objet, un procédé cosmétique pour limiter la migration d'une composition cosmétique contenant une phase grasse liquide, consistant à structurer ladite phase grasse par une quantité suffisante d'au moins un polymère de masse moléculaire moyenne en poids allant de 1000 à 30 000, comportant a) un squelette polymérique, ayant des motifs de répétition hydrocarbonés pourvus d'au moins un hétéroatome, et b) au moins une chaîne grasse pendante et/ou au moins une chaîne grasse terminale, éventuellement fonctionnalisées, ayant de 12 à 120 atomes de carbone, liées à ces motifs, les chaînes grasses représentant de 40 à 98 % du nombre total des motifs à hétéroatome et des chaînes grasses.

L'invention a encore pour objet un stick de maquillage de la peau, des lèvres et/ou des phanères, et en particulier des lèvres, contenant au moins un pigment en quantité suffisante pour maquiller la peau, les lèvres et/ou les phanères et une phase grasse continue liquide, structurée par au moins un polymère de masse moléculaire moyenne en poids allant de 1 000 à 30 000, comportant a) un squelette polymérique, ayant des motifs de répétition hydrocarbonés pourvus d'au moins un hétéroatome, et b) au moins une chaîne grasse pendante et/ou au moins une chaîne grasse terminale ayant de 12 à 120 atomes de carbone, liées à ces motifs, les chaînes grasses représentant de 40 à 98 % du nombre total des motifs à hétéroatome et des chaînes grasses, le pigment, la phase grasse et le polymère formant un milieu physiologiquement acceptable, la composition comprenant, en outre, au moins un composé amphiphile liquide à température ambiante, de valeur de HLB inférieur à 12.

L'invention est illustrée plus en détail dans les exemples suivants. Les pourcentages sont donnés en poids.

### Exemple 1 : Rouge à lèvres

- Uniclear 80 25,0 %
- Huile de parléam 56,0 %
- Polyglycéryl-2 polyhydroxystéarate 10,0 %
- Pigments (oxyde de fer brun + oxyde de titane) 9,0 %
*Préparation* : On solubilise (ou dissous) l'Uniclear 80 grâce au polyglycéryl-2 polyhydroxystéarate dans l'huile de parléam, à 100 °C, puis on ajout les pigments. L'ensemble est mélangé à l'aide d'une turbine défloculeuse (Raynerie) puis coulé dans des moules de rouge à lèvres.

On obtient un stick de rouge à lèvres ayant une dureté de 425 g mesurée à l'aide de l'analyseur de texture TA-XT2 à 20 °C. Le rouge à lèvres obtenu est brillant et non migrant. Ceci a été confirmé par un test sur un panel d'experts en comparaison avec un produit brillant de l'art antérieur Rouge Absolu de Lancôme. Le rouge à lèvres de l'invention a été jugée plus brillant à l'application que celui de l'art antérieur pour l'ensemble des testeurs et moins migrant au bout de 2 heures de port.

### Exemple 2 : Fard à paupières anhydre

- Uniclear 80 25,0 %
- Huile de parléam 35,1 %
- Oléate de glycéryle 31.25 %
- Pigments qsp 100 %

Ce fard à paupières sous forme de stick a été réalisé comme dans l'exemple 1. Il est brillant et non migrant.

### Exemple 3 : Rouge à lèvres

II se différencie de celui de l'exemple 1 par l'emploi de l'Uniclear 100 au lieu de l'Uniclear 80.

### Contre exemple

On a reproduit l'exemple 1 de rouges à lèvres en remplaçant le polyamide Uniclear 80 par le polyamide Versamid® 930 vendu par la société Henkel, puis par le polyamide Macromelt® 6212 vendu aussi par la société Henkel, ces deux polyamides étant exempts de groupement terminal à chaîne alkyle ou alcényle à au moins 4 atomes de carbone, lié au squelette polyamide par un groupe ester.

Les produits obtenus sont totalement hétérogènes et sous forme bi-phasée. Ils n'ont nullement l'aspect et la dureté d'un stick.

## Revendications

1. Composition structurée contenant au moins une matière colorante, une phase grasse continue liquide, structurée par au moins un polymère de masse moléculaire moyenne en poids allant de 1 000 à 30 000, comportant a) un squelette polymérique, ayant des motifs de répétition hydrocarbonés pourvus d'au moins un hétéroatome, et b) au moins une chaîne grasse pendante et/ou au moins une chaîne grasse terminale, éventuellement fonctionnalisées, ayant de 12 à 120 atomes de carbone, liées à ces motifs, les chaînes grasses représentant de 40 à 98 % du nombre total des motifs à hétéroatome et des chaînes grasses, ladite composition se présentant sous forme d'un solide, exempte de cire, la matière colorante, la phase grasse liquide et le polymère formant un milieu physiologiquement acceptable, la composition comprenant en outre, au moins un composé amphiphile liquide à température ambiante, de valeur de HLB inférieur à 12.

2. Composition selon la revendication 1, **caractérisée en ce qu'**elle est autoportée.

3. Composition selon la revendication 1 ou 2, **caractérisée en ce qu'**elle se présente sous forme d'un solide de dureté allant de 0,19 à 19,6 N (20 à 2 000 gf) et de préférence de 0,19 à 8,82 N (20 à 900 gf).

4. Composition selon l'une des revendications précédentes, **caractérisée en ce que** les chaînes grasses représentent de 50 à 95 % du nombre total des motifs à hétéroatome et des chaînes grasses.

5. Composition selon l'une des revendications précédentes, **caractérisée en ce que** les motifs à hétéroatome sont des amides.

6. Composition selon l'une des revendications précédentes, **caractérisée en ce que** les chaînes grasses pendantes sont liées directement à l'un au moins desdits hétéroatomes.

7. Composition selon l'une des revendications précédentes, **caractérisée en ce que** les chaînes grasses terminales sont liées au squelette par des groupes ester.

8. Composition selon l'une des revendications précédentes, **caractérisée en ce que** les chaînes grasses ont de 12 à 68 atomes de carbone.

9. Composition selon l'une des revendications précédentes, **caractérisée en ce que** le polymère est choisi parmi les polymères de formule (I) suivante et leurs mélanges : 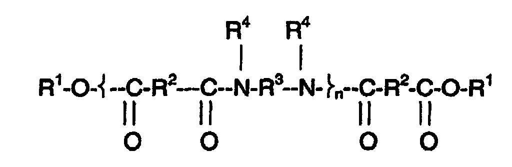 dans laquelle n désigne un nombre de motifs amide tel que le nombre de groupes ester représente de 10 % à 50 % du nombre total des groupes ester et amide ; R¹ est à chaque occurrence indépendamment un groupe alkyle ou alcényle ayant au moins 4 atomes de carbone ; R² représente à chaque occurrence Indépendamment un groupe hydrocarboné en C₄ à C₄₂ à condition que 50 % au moins des groupes R² représentent un groupe hydrocarboné en C₃₀ à C₄₂; R³ représente à chaque occurrence indépendamment un groupe organique pourvus d'au moins 2 atomes de carbone, d'atomes d'hydrogène et optionnellement d'un ou plusieurs atomes d'oxygène ou d'azote ; et R⁴ représente à chaque occurrence indépendamment un atome d'hydrogène, un groupe alkyle en C₁ à C₁₀ ou une liaison directe à R³ ou un autre R⁴ de sorte que l'atome d'azote auquel sont liés à la fois R³ et R⁴ fasse partie d'une structure hétérocyclique définie par R⁴-N-R³, avec au moins 50 % des R⁴ représentant un atome d'hydrogène.

10. Composition selon la revendication précédente, **caractérisée en ce que** R¹ est un groupe alkyle en C₁₂ à C₂₂.

11. Composition selon l'une des revendications 9 ou 10, **caractérisée en ce que** R² sont des groupes ayant de 30 à 42 atomes de carbone.

12. Composition selon l'une des revendications précédentes, **caractérisée en ce que** le composé amphiphile présente une valeur de HLB allant de 1 à 7 et de préférence de 1 à 5.

13. Composition selon l'une des revendications précédentes, **caractérisée en ce que** le composé amphiphile comprend une partie lipophile liée à une partie polaire, la partie lipophile comportant une chaîne carbonée ayant au moins 8 atomes de carbone, de préférence de 16 à 32 atomes de carbone et mieux de 18 à 28 atomes de carbone.

14. Composition selon la revendication précédente, **caractérisée en ce que** la partie polaire est le reste d'un composé choisi parmi les alcools et les polyols ayant de 1 à 12 groupements hydroxyle, les polyoxyalkylènes comportant au moins 2 motifs oxyalkylénés et ayant de 0 à 20 motifs oxypropylénés et/ou de 0 à 20 motifs oxyéthylénés.

15. Composition selon l'une des revendicationsprécédentes, **caractérisée en ce que** le composé amphiphile est choisi parmi les hydroxystéarates, les oléates, les isostéarates du glycérol, du sorbitan ou du méthylglucose, l'octyldodécanol.

16. Composition selon l'une des revendications précédentes, **caractérisée en ce que** le composé amphiphile représente de 0,1 à 35 % du poids total de la composition et mieux de 2 à 15%.

17. Composition selon l'une dès revendications précédentes, **caractérisée en ce que** le polymère représente de 0,5 à 80 % du poids total de la composition et mieux de 5 à 40 %.

18. Composition selon l'une des revendications précédentes, **caractérisée en ce que** le polymère comprend une masse moléculaire moyenne en poids allant de 1 000 à 10 000 et mieux de 2 000 à 8 000.

19. Composition selon l'une des revendications précédentes, **caractérisée en ce que** la phase grasse liquide contient plus de 40 % et mieux plus de 50 % d'huile(s) liquide(s) ayant un groupement similaire à celui des motifs à hétéroatome.

20. Composition selon l'une des revendications 5 à 19, **caractérisée en ce que** la phase grasse liquide contient plus de 40 % et mieux plus de 50 % d'huile ou mélange d'huiles liquides apolaires.

21. Composition selon l'une des revendications précédentes, **caractérisée en ce que** la phase grasse contient au moins une huile hydrocarbonée d'origine minérale ou synthétique.

22. Composition selon l'une des revendications précédentes, **caractérisée en ce que** la phase grasse contient au moins une huile apolaire choisie parmi l'huile de parléam, les isoparaffines, le squalane et leurs mélanges.

23. Composition selon l'une des revendications précédentes, **caractérisée en ce que** la phase grasse liquide représente de 5 à 99 % du poids total de la composition et mieux de 20 à 75 %.

24. Composition selon l'une des revendications précédentes, **caractérisée en ce qu'**elle constitue une composition de soin et/ou de traitement et/ou de maquillage des matières kératiniques.

25. Composition selon l'une des revendications précédentes, **caractérisée en ce qu'**elle contient, en outre, au moins un actif cosmétique ou dermatologique.

26. Composition selon l'une des revendications précédentes, **caractérisée en ce qu'**elle contient au moins un additif choisi parmi l'eau, les antioxydants, les huiles essentielles, les conservateurs, les neutralisants, les polymères liposolubles, les charges, les parfums et leurs mélanges.

27. Composition selon l'une des revendications précédentes, **caractérisée en ce qu'**elle se présente sous forme d'un gel rigide anhydre transparent, et notamment de stick anhydre transparent.

28. Composition selon l'une des revendications précédentes, **caractérisée en ce que** la matière colorante est choisie parmi les colorants lipophiles, les colorants hydrophiles, les pigments, les nacres et leurs mélanges.

29. Composition selon l'une des revendications précédentes, **caractérisée en ce que** la matière colorante est présente à raison de 0,01 à 40 % du poids total de la composition, de préférence de 5 à 25 %.

30. Composition structurée de maquillage de la peau, des lèvres et/ou des phanères, contenant au moins un pigment en quantité suffisante pour maquiller la peau, les lèvres et/ou les phanères et une phase grasse continue liquide, structurée par au moins un polymère de masse moléculaire moyenne en poids allant de 1 000 à 30 000, comportant a) un squelette polymérique, ayant des motifs de répétition hydrocarbonés pourvus d'au moins un hétéroatome, et b) au moins une chaîne grasse pendante et/ou au moins une chaîne grasse terminale ayant de 12 à 120 atomes de carbone, liées à ces motifs, les chaînes grasses représentant de 40 à 98 % du nombre total des motifs à hétéroatome et des chaînes grasses, ladite composition se présentant sous forme d'un solide, le pigment, la phase grasse liquide et le polymère formant un milieu physiologiquement acceptable, la composition comprenant, en outre, au moins un composé amphiphile liquide à température ambiante, de valeur de HLB inférieur à 12.

31. Composition selon la revendication 30, **caractérisée en ce qu'**elle est autoportée.

32. Composition selon la revendication 30, **caractérisée en ce qu'**elle se présente sous forme d'un solide de dureté allant de 0,19 à 19,6 N (20 à 2 000 gf) et de préférence de 0,19 à 8,82 N (20 à 900 gf).

33. Composition selon la revendication 32, **caractérisée en ce que** le polymère est un polyamide.

34. Composition selon l'une des revendications 32 à 33, **caractérisée en ce que** la ou les chaînes grasses terminales sont liées au squelette carboné par des groupes ester.

35. Composition selon l'une des revendications 1 à 32, **caractérisée en ce qu'**elle se présente sous forme de mascara pain, d'eye liner, de fond de teint, de rouge à lèvres, de blush, de produit déodorant ou démaquillant, de produit de maquillage du corps, de fard à paupières ou à joues, de produit anti-cerne.

36. Composition selon l'une des revendications précédentes, **caractérisée en ce que** les motifs à hétéroatome comprennent chacun de un à plusieurs hétéroatomes choisis parmi les atomes d'azote, de soufre, de phosphore et leurs associations, associés éventuellement à un ou plusieurs atome d'oxygène.

37. Composition selon l'une des revendications précédentes, **caractérisée en ce que** le polymère structurant de la composition a une température de ramollissement supérieure à 70°C et pouvant aller jusqu'à 190°C, de préférence une température de ramollissement allant de 80 à 130°C et mieux de 80°C à 105°C.

38. Composition selon la revendication 30 ou 31, ladite composition se trouvant par exemple sous forme d'un solide autoporté, sous la forme d'un rouge à lèvres.

39. Composition selon la revendication 30 on 31, sous la forme d'un stick de maquillage de la peau, des lèvres et/ou des phanères, et en particulier des lèvres.

40. Procédé cosmétique de soin, de maquillage ou de traitement des matières kératiniques des êtres humains, comprenant l'application sur les matières kératiniques d'une composition cosmétique conforme à l'une des revendications précédentes.

41. Utilisation d'une quantité suffisante d'au moins un polymère de masse moléculaire moyenne en poids allant de 1000 à 30 000, comportant a) un squelette polymérique ayant des motifs de répétition hydrocarbonés pourvus d'au moins un hétéroatome, et b) au moins une chaîne grasse pendante et/ou au moins une chaîne grasse terminale, éventuellement fonctionnalisées, ayant de 12 à 120 atomes de carbone, liées à ces motifs, ces chaînes grasses représentant de 40 à 98 % du nombre total des motifs à hétéroatome et des chaînes grasses, dans une composition cosmétique non thérapeutique ou pour la fabrication d'une composition physiologiquement acceptable, contenant une phase grasse continue liquide et une matière colorante, pour structurer ladite composition sous forme d'un solide autoporté de dureté allant de allant de 0,19 à 19,6 N (20 à 2 000 gf), la composition comprenant en outre, au moins un composé amphiphile liquide à température ambiante, de valeur de HLB inférieur à 12, ladite composition étant exempte de cire.

42. Utilisation selon la revendication 41, dans une composition cosmétique non thérapeutique ou pour la fabrication d'une composition physiologiquement acceptable, pour structurer ladite composition sous forme d'un solide autoporté de dureté allant de 0,19 à 8,82 N (20 à 900 gf), et mieux de 0,19 à 5,88 N (20 à 600 gf).

43. Utilisation selon la revendication 41 ou 42, **caractérisée en ce que** te polymère est un polyamide comportant des groupements terminaux à fonction ester comportant une chaîne hydrocarbonée ayant de 10 à 42 atomes de carbone.

44. Utilisation selon l'une des revendications 41 à 43, **caractérisée en ce que** le polymère est associé à un composé amphiphile de valeur de HLB allant de 1 à 7 et de préférence de 1 à 5.

45. Utilisation d'une phase grasse liquide continue, structurée essentiellement par une quantité suffisante d'au moins un polymère de masse moléculaire moyenne en poids allant de 1000 à 30 000, comportant a) un squelette polymérique, ayant des motifs de répétition hydrocarbonés pourvus d'au moins un hétéroatome, et b) au moins une chaîne grasse pendante et/ou au moins une chaîne grasse terminale, éventuellement fonctionnalisées , ayant de 12 à 120 atomes de carbone, liées à ces motifs, les chaînes grasses représentant de 40 à 98 % du nombre total des motifs à hétéroatome et des chaînes grasses, dans une composition cosmétique non thérapeutique ou pour la fabrication d'une composition physiologiquement acceptable, contenant un composé amphiphile liquide de valeur de HLB inférieure à 12 et notamment allant de 1 à 7 de préférence de 1 à 5, ladite composition étant rigide, notamment autoportée, exempte de cire, brillante et/ou non migrante.

46. Utilisation selon la revendication précédente, dans laquelle le polymère est un polyamide comportant des groupements terminaux à fonction ester comportant une chaîne hydrocarbonée ayant de 10 à 42 atomes de carbone.

47. Utilisation d'une phase grasse liquide continue, structurée essentiellement par une quantité suffisante d'au moins un polymère de masse moléculaire moyenne en poids allant de 1000 à 30 000, comportant a) un squelette polymérique, ayant des motifs de répétition hydrocarbonés pourvus d'au moins un hétéroatome, et b) au moins une chaîne grasse pendante et/ou au moins une chaîne grasse terminale, éventuellement fonctionnalisées, ayant de 12 à 120 atomes de carbone, liées à ces motifs, les chaînes grasses représentant de 40 à 98 % du nombre total des motifs à hétéroatome et des chaînes grasses, dans une composition cosmétique non thérapeutique ou pour la fabrication d'une composition physiologiquement acceptable, comme agent pour limiter la migration de ladite composition.

48. Utilisation selon l'une des revendications 45 à 47, dans laquelle la composition a une dureté allant de 0,19 à 19,6 N (20 à 2 000 gf) et de préférence de 0,19 à 8,82 N (20 à 900 gf), et mieux de 0,19 à 5,88 N (20 à 600 gf).

49. Procédé cosmétique pour limiter la migration d'une composition cosmétique contenant une phase grasse liquide, consistant à structurer ladite phase grasse par une quantité suffisante d'au moins un polymère de masse moléculaire moyenne en poids allant de 1000 à 30 000, comportant a) un squelette polymérique, ayant des motifs de répétition hydrocarbonés pourvus d'au moins un hétéroatome, et b) au moins une chaîne répétition hydrocarbonés pourvus d'au moins un hétéroatome, et b) au moins une chaîne grasse pendante et/ou au moins une chaîne grasse terminale, éventuellement fonctionnalisées, ayant de 12 à 120 atomes de carbone, liées à ces motifs, les chaînes grasses représentant de 40 à 98 % du nombre total des motifs à hétéroatome et des chaînes grasses.

## Claims

1. Structured composition containing at least one dyestuff and a continuous liquid fatty phase, structured with at least one polymer with a weight-average molecular mass ranging from 1000 to 30,000, comprising a) a polymeric skeleton, having repeating hydrocarbon-based units containing at least one hetero atom, and b) at least one pendant fatty chain and/or at least one terminal fatty chain, which are optionally functionalized, containing from 12 to 120 carbon atoms, linked to these units, the fatty chains representing from 40% to 98% of the total number of units containing a hetero atom and fatty chains, the said composition being in the form of a wax-free solid, the dyestuff, the liquid fatty phase and the polymer forming a physiologically acceptable medium, the composition also comprising at least one amphiphilic compound which is liquid at room temperature, with an HLB value of less than 12.

2. Composition according to Claim 1, **characterized in that** it is self-supporting.

3. Composition according to Claim 1 or 2, **characterized in that** it is in the form of a solid. having a hardness ranging from 0.19 to 19.6 N (20 gf to 2000 gf) and preferably from 0.19 to 8.82 N (20 gf to 900 gf).

4. Composition according to one of the preceding claims, **characterized in that** the fatty chains represent from 50% to 95% of the total number of units containing a hetero atom and fatty chains.

5. Composition according to one of the preceding claims, **characterized in that** the units containing a hetero atom are amides.

6. Composition according to one of the preceding claims, **characterized in that** the pendant fatty chains are linked directly to at least one of the said hetero atoms.

7. Composition according to one of the preceding claims, **characterized in that** the terminal fatty chains are linked to the skeleton via ester groups.

8. Composition according to one of the preceding claims, **characterized in that** the fatty chains contain from 12 to 68 carbon atoms.

9. Composition according to one of the preceding claims, **characterized in that** the polymer is chosen from the polymers of formula (I) below, and mixtures thereof: 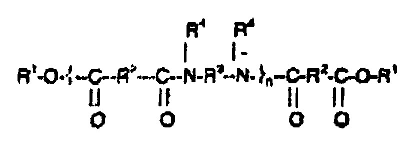 in which n denotes a number of amide units such that the number of ester groups represents from 10% to 50% of the total number of ester and amide groups; R¹ is independently, at each occurrence, an alkyl or alkenyl group containing at least 4 carbon atoms; R² independently represents, at each occurrence, a C₄ to C₄₂ hydrocarbon-based group on condition that at least 50% of the groups R² represent a C₃₀ to C₄₂ hydrocarbon-based group; R³ independently represents, at each occurrence, an organic group containing at least 2 carbon atoms, hydrogen atoms and optionally one or more oxygen or nitrogen atoms; and R⁴ independently represents, at each occurrence, a hydrogen atom, a C₁ to C₁₀ alkyl group or a direct bond to R³ or to another R⁴ such that the nitrogen atom to which both R³ and R⁴ are linked forms part of a heterocyclic structure defined by R⁴-N-R³, with at least 50% of the groups R⁴ representing a hydrogen atom.

10. Composition according to the preceding claim, **characterized in that** R¹ is a C₁₂ to C₂₂ alkyl group.

11. Composition according to either of Claims 9 and 10, **characterized in that** the groups R² are groups containing from 30 to 42 carbon atoms.

12. Composition according to one of the preceding claims, **characterized in that** the amphiphilic compound has an HLB value ranging from 1 to 7 and preferably from 1 to 5.

13. Composition according to one of the preceding claims, **characterized in that** the amphiphilic compound comprises a lipophilic part linked to a polar part, the lipophilic part comprising a carbon-based chain containing at least 8 carbon atoms, preferably from 16 to 32 carbon atoms and better still from 18 to 28 carbon atoms.

14. Composition according to the preceding claim, **characterized in that** the polar part is the residue of a compound chosen from alcohols and polyols containing from 1 to 12 hydroxyl groups or polyoxyalkylene groups comprising at least 2 oxyalkylene units and containing from 0 to 20 oxypropylene units and/or from 0 to 20 oxyethylene units.

15. Composition according to one of the preceding claims, **characterized in that** the amphiphilic compound is chosen from the hydroxystearates, oleates and isostearates of glycerol, of sorbitan or of methylglucose and octyldodecanol.

16. Composition according to one of the preceding claims, **characterized in that** the amphiphilic compound represents from 0.1% to 35% of the total weight of the composition and better still from 2% to 15%.

17. Composition according to one of the preceding claims, **characterized in that** the polymer represents from 0.5% to 80% of the total weight of the composition and better still from 5% to 40%.

18. Composition according to one of the preceding claims, **characterized in that** the polymer comprises a weight-average molecular mass ranging from 1000 to 10,000 and better still from 2000 to 8000.

19. Composition according to one of the preceding claims, **characterized in that** the liquid fatty phase contains more than 40% and better still more than 50% of liquid oil(s) containing a group similar to that of the units containing a hetero atom.

20. Composition according to one of Claims 5 to 19, **characterized in that** the liquid fatty phase contains more than 40% and better still more than 50% of apolar liquid oil or mixture of oils.

21. Composition according to one of the preceding claims, **characterized in that** the fatty phase contains at least one hydrocarbon-based oil of mineral or synthetic origin.

22. Composition according to one of the preceding claims, **characterized in that** the fatty phase contains at least one apolar oil chosen from parleam oil, isoparaffins and squalane, and mixtures thereof.

23. Composition according to one of the preceding claims, **characterized in that** the liquid fatty phase represents from 5% to 99% of the total weight of the composition and better still from 20% to 75%.

24. Composition according to one of the preceding claims, **characterized in that** it constitutes a care and/or treating and/or make-up composition for keratin materials.

25. Composition according to one of the preceding claims, **characterized in that** it also contains at least one cosmetic or dermatological active agent.

26. Composition according to one of the preceding claims, **characterized in that** it contains at least one additive chosen from water, antioxidants, essential oils, preserving agents, neutralizing agents, liposoluble polymers, fillers and fragrances, and mixtures thereof.

27. Composition according to one of the preceding claims, **characterized in that** it is in the form of a transparent anhydrous rigid gel and in particular in the form of a transparent anhydrous stick.

28. Composition according to one of the preceding claims, **characterized in that** the dyestuff is chosen from lipophilic dyes, hydrophilic dyes, pigments and nacres, and mixtures thereof.

29. Composition according to one of the preceding claims, **characterized in that** the dyestuff is present in a proportion of from 0.01% to 40% relative to the total weight of the composition, preferably from 5% to 25%.

30. Structured make-up composition for the skin, the lips and/or superficial body growths, containing at least one pigment in an amount which is sufficient to make up the skin, the lips and/or superficial body growths and a liquid continuous fatty phase, structured with at least one polymer with a weight-average molecular mass ranging from 1000 to 30,000, comprising a) a polymeric skeleton, having repeating hydrocarbon-based units containing at least one hetero atom, and b) at least one pendant fatty chain and/or at least one terminal fatty chain, containing from 12 to 120 carbon atoms, linked to these units, the fatty chains representing from 40% to 98% of the total number of units containing a hetero atom and fatty chains, the said composition being in the form of a solid, the pigment, the liquid fatty phase and the polymer forming a physiologically acceptable medium, the composition also comprising at least one amphiphilic compound which is liquid at room temperature, with an HLB value of less than 12.

31. Composition according to Claim 30, **characterized in that** it is self-supporting.

32. Composition according to Claim 30, **characterized in that** it is in the form of a solid having a hardness ranging from 0.19 to 19.6 N (20 gf to 2000 gf), preferably from 0.19 to 8.82 N (20 gf to 900 gf).

33. Composition according to Claim 32, **characterized in that** the polymer is a polyamide.

34. Composition according to either of Claims 32 and 33, **characterized in that** the terminal fatty chain(s) is(are) linked to the carbon-based skeleton via ester groups.

35. Composition according to one of Claims 1 to 32, **characterized in that** it is in the form of a tablet of mascara, an eyeliner, a foundation, a lipstick, a blusher, a deodorant product, a make-up-removing product; a product for making up the body, an eyeshadow, a face powder or a concealer product.

36. Composition according to one of the preceding claims, **characterized in that** the units containing a hetero atom each comprise one or more hetero atoms chosen from nitrogen, sulphur and phosphorus atoms and combinations thereof, optionally combined with one or more oxygen atoms.

37. Composition according to one of the preceding claims, **characterized in that** the structuring polymer of the composition has a softening point of greater than 70°C and which may be up to 190°C, preferably a softening point ranging from 80 to 130°C and better still from 80°C to 105°C.

38. Composition according to Claim 30 or 31, the said composition being, for example, in the form of a self-supporting solid or in the form of a lipstick.

39. Composition according to Claim 30 or 31, in the form of a make-up stick for the skin, the lips and/or superficial body growths, and in particular for the lips.

40. Cosmetic process for caring for, making up or treating keratin materials of human beings, comprising the application of a cosmetic composition in accordance with one of the preceding claims to the keratin materials.

41. Use of a sufficient amount of at least one polymer with a weight-average molecular mass ranging from 1000 to 30,000, comprising a) a polymeric skeleton with hydrocarbon-based repeating units containing at least one hetero atom, and b) at least one pendant fatty chain and/or at least one terminal fatty chain, which are optionally functionalized, containing from 12 to 120 carbon atoms, linked to these units, these fatty chains representing from 40% to 98% of the total number of units containing a hetero atom and fatty chains, in a non therapeutic cosmetic composition or for the manufacture of a physiologically acceptable composition, which contains a liquid continuous fatty phase and a dyestuff, to structure the said composition in the form of a self-supporting solid having a hardness ranging from 0.19 to 19.6 N (20 gf to 2000 gf), the composition also comprising at least one amphiphilic compound which is liquid at room temperature, with an HLB value of less than 12, the said composition being wax-free.

42. Use according to Claim 41, in a non therapeutic cosmetic composition or for the manufacture of a physiologically acceptable composition, to structure the said composition in the form of a self-supporting solid having a hardness ranging from 0.19 to 8.82 N (20gf to 900 gf) and better still from 0.19 to 5.88 N (20gf to 600 gf).

43. Use according to Claim 41 or 42, **characterized in that** the polymer is a polyamide comprising end groups containing an ester function comprising a hydrocarbon-based chain containing from 10 to 42 carbon atoms.

44. Use according to one of Claims 41 to 43, **characterized in that** the polymer is combined with an amphiphilic compound with an HLB value ranging from 1 to 7 and preferably from 1 to 5.

45. Use of a liquid continuous fatty phase, which is essentially structured with a sufficient amount of at least one polymer with a weight-average molecular mass ranging from 1000 to 30,000, comprising a) a polymeric skeleton with hydrocarbon-based repeating units containing at least one hetero atom, and b) at least one pendant fatty chain and/or at least one terminal fatty chain, which are optionally functionalized, containing from 12 to 120 carbon atoms, linked to these units, the fatty chains representing from 40% to 98% of the total number of units containing a hetero atom and fatty chains, in a non therapeutic cosmetic composition or for the manufacture of a physiologically acceptable composition, containing a lipid amphiphilic compound with an HLB value of less than 12 and in particular ranging from 1 to 7 and preferably from 1 to 5, the said composition being rigid, in particular self-supporting, wax-free, glossy and/or non-migrating.

46. Use according to the preceding claim, in which the polymer is a polyamide comprising end groups containing an ester function comprising a hydrocarbon-based chain containing from 10 to 42 carbon atoms.

47. Use of a liquid continuous fatty phase, which is essentially structured with a sufficient amount of at least one polymer with a weight-average molecular mass ranging from 1000 to 30,000, comprising a) a polymeric skeleton with hydrocarbon-based repeating units containing at least one hetero atom, and b) at least one pendant fatty chain and/or at least one terminal fatty chain, which are optionally functionalized, containing from 12 to 120 carbon atoms, linked to these units, the fatty chains representing from 40% to 98% of the total number of units containing a hetero atom and fatty chains, in a non therapeutic cosmetic composition or for the manufacture of a physiologically acceptable composition, as an agent for limiting the migration of the said composition.

48. Use according to one of Claims 45 to 47, in which the composition has a hardness ranging from 0.19 to 19.6 N (20 gf to 2000 gf), preferably from 0.19 to 8.82 N (20 gf to 900 gf) and better still from 0.19 to 5.88 N (20 gf to 600 gf).

49. Cosmetic process for limiting the migration of a cosmetic composition containing a liquid fatty phase, which consists in structuring the said fatty phase with a sufficient amount of at least one polymer with a weight-average molecular mass ranging from 1000 to 30,000, comprising a) a polymeric skeleton with hydrocarbon-based repeating units containing at least one hetero atom, and b) at least one pendant fatty chain and/or at least one terminal fatty chain, which are optionally functionalized, containing from 12 to 120 carbon atoms, linked to these units, the fatty chains representing from 40% to 98% of the total number of units containing a hetero atom and fatty chains.

## Patentansprüche

1. Strukturierte Zusammensetzung, die mindestens ein Farbmittel. und eine kontinuierliche flüssige Fettphase enthält, die mit mindestens einem Polymer mit einem Gewichtsmittel des Molekulargewichts von 1 000 bis 30 000 strukturiert ist, das a) ein Polymergerüst mit wiederkehrenden Kohlenwasserstoffeinheiten, die mindestens ein Heteroatom enthalten, und b) mindestens eine Fettkette als Seitenkette mit 12 bis 120 Kohlenstoffatomen und/oder mindestens eine endständige Fettkette mit 12 bis 120 Kohlenstoffatomen aufweist, die gegebenenfalls funktionalisiert sind und die an diese Einheiten gebunden sind, wobei die Fettketten 40 bis 98 % der Gesamtzahl der Einheiten mit Heteroatom und der Fettketten ausmachen, wobei die Zusammensetzung in Form eines wachsfreien Feststoffs vorliegt, wobei das Farbmittel, die flüssige Fettphase und das Polymer ein physiologisch akzeptables Medium bilden und wobei die Zusammensetzung ferner mindestens eine bei Raumtemperatur flüssige, amphiphile Verbindung mit einem HLB-Wert unter 12 enthält.

2. Zusammensetzung nach Anspruch 1, **dadurch gekennzeichnet, daß** sie selbsttragend ist.

3. Zusammensetzung nach Anspruch 1 oder 2, **dadurch gekennzeichnet, daß** sie als Feststoff mit einer Härte im Bereich von 0,19 bis 19,6 N (20 bis 2000 gf) und vorzugsweise 0,19 bis 8,82 N (20 bis 900 gf) vorliegt.

4. Zusammensetzung nach einem der vorhergehenden Ansprüche, **dadurch gekennzeichnet, daß** die Fettketten 50 bis 95 % der Gesamtzahl der Einheiten mit Heteroatom und der Fettketten ausmachen.

5. Zusammensetzung nach einem der vorhergehenden Ansprüche, **dadurch gekennzeichnet, daß** die Einheiten mit Heteroatom Amide sind.

6. Zusammensetzung nach einem der vorhergehenden Ansprüche, **dadurch gekennzeichnet, daß** die als Seitenketten vorliegenden Fettketten direkt an mindestens eines der Heteroatome gebunden sind.

7. Zusammensetzung nach einem der vorhergehenden Ansprüche, **dadurch gekennzeichnet, daß** die endständigen Fettketten über eine Estergruppe an das Gerüst gebunden sind.

8. Zusammensetzung nach einem der vorhergehenden Ansprüche, **dadurch gekennzeichnet, daß** die Fettketten 12 bis 68 Kohlenstoffatome aufweisen.

9. Zusammensetzung nach einem der vorhergehenden Ansprüche, **dadurch gekennzeichnet, daß** das Polymer unter den Polymeren der folgenden Formel (I) und deren Gemischen ausgewählt ist: 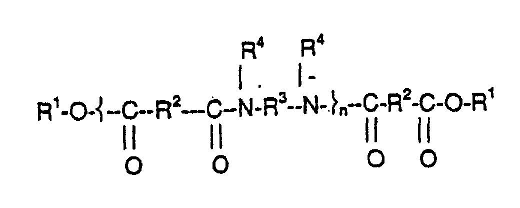 wobei n die Anzahl der Amideinheiten bezeichnet und so ausgewählt ist, daß die Anzahl der Estergruppen 10 bis 50 % der Gesamtzahl der Ester- und Amidgruppen ausmacht; die Gruppen R¹ jeweils unabhängig voneinander Alkyl- oder Alkenylgruppen mit mindestens 4 Kohlenstoffatomen bedeuten; die Gruppen R² jeweils unabhängig voneinander C₄₋₄₂-Kohlenwasserstoffgruppen bedeuten, mit der Maßgabe, daß mindestens 50 % der Gruppen R² C₃₀₋₄₂-Kohlenwasserstoffgruppen sind; die Gruppen R³ jeweils unabhängig voneinander organische Gruppen bedeuten, die mindestens zwei Kohlenstoffatome, Wasserstoffatome und gewünschtenfalls ein oder mehrere Sauerstoffatome oder Stickstoffatome enthalten; und die Gruppen R⁴ jeweils unabhängig voneinander Wasserstoff, C₁₋₁₀-Alkyl oder eine direkte Bindung zu R³ oder einer weiteren Gruppe R⁴ bedeuten, die so gebildet wird, daß das Stickstoffatom, an das die Gruppen R³ und R⁴ gleichzeitig gebunden sind, Teil einer heterocyclischen Struktur ist, die durch R⁴-N-R³ definiert ist, wobei mindestens 50 % der Gruppen R⁴ Wasserstoff bedeuten.

10. Zusammensetzung nach dem vorhergehenden Anspruch, **dadurch gekennzeichnet, daß** R¹ eine C₁₂₋₂₂-Alkylgrppe ist.

11. Zusammensetzung nach einem der Ansprüche 9 oder 10, **dadurch gekennzeichnet, daß** die Gruppen R² Gruppen mit 30 bis 42 Kohlenstoffatomen sind.

12. Zusammensetzung nach einem der vorhergehenden Ansprüche, **dadurch gekennzeichnet, daß** die amphiphile Verbindung einen HLB-Wert von 1 bis 7 und vorzugsweise 1 bis 5 aufweist.

13. Zusammensetzung nach einem der vorhergehenden Ansprüche, **dadurch gekennzeichnet, daß** die amphiphile Verbindung einen lipophilen Bereich aufweist, der an einen polaren Bereich gebunden ist, wobei der lipophile Bereich eine kohlenstoffhaltige Kette mit mindestens 8 Kohlenstoffatomen und vorzugsweise 16 bis 32 Kohlenstoffatomen und besser 18 bis 28 Kohlenstoffatomen enthält.

14. Zusammensetzung nach dem vorhergehenden Anspruch, **dadurch gekennzeichnet, daß** der polare Bereich der Rest einer Verbindung ist, die unter den Alkoholen und Polyolen mit 1 bis 12 Hydroxygruppen und den Polyoxyalkylenen ausgewählt ist, die mindestens 2 Alkylenoxideinheiten aufweisen und 0 bis 20 Propylenoxideinheiten und/oder 0 bis 20 Ethylenoxideinheiten tragen.

15. Zusammensetzung nach einem der vorhergehenden Ansprüche, **dadurch gekennzeichnet, daß** die amphiphile Verbindung unter den Hydroxystearaten, Oleaten, Isostearaten von Glycerin, Sorbitan oder Methylglucose und Octyldodecanol ausgewählt ist.

16. Zusammensetzung nach einem der vorhergehenden Ansprüche, **dadurch gekennzeichnet, daß** die amphiphile Verbindung 0,1 bis 35 % und besser 2 bis 15 % des Gesamtgewichts der Zusammensetzung ausmacht.

17. Zusammensetzung nach einem der vorhergehenden Ansprüche, **dadurch gekennzeichnet, daß** das Polymer 0,5 bis 80 % des Gesamtgewichts der Zusammensetzung und besser 5 bis 40 % ausmacht.

18. Zusammensetzung nach einem der vorhergehenden Ansprüche, **dadurch gekennzeichnet, daß** das Polymer ein Gewichtsmittel des Molekulargewichts von 1 000 bis 10 000 und besser 2 000 bis 8 000 aufweist.

19. Zusammensetzung nach einem der vorhergehenden Ansprüche, **dadurch gekennzeichnet, daß** die flüssige Fettphase mehr als 40 % und besser mehr als 50 % flüssige(s) Öl(e) enthält, die eine Gruppe aufweisen, die den Einheiten mit Heteroatom ähnelt.

20. Zusammensetzung nach einem der Ansprüche 5 bis 19, **dadurch gekennzeichnet, daß** die flüssige Fettphase mehr als 40 % und besser mehr als 50 % eines apolaren flüssigen Öls oder Gemisches von apolaren flüssigen Ölen enthält.

21. Zusammensetzung nach einem der vorhergehenden Ansprüche, **dadurch gekennzeichnet, daß** die Fettphase mindestens ein Kohlenwasserstofföl mineralischer oder synthetischer Herkunft enthält.

22. Zusammensetzung nach einem der vorhergehenden Ansprüche, **dadurch gekennzeichnet, daß** die Fettphase mindestens ein apolares Öl enthält, das unter Parleam, Isoparaffinen, Squalan und ihren Gemischen ausgewählt ist.

23. Zusammensetzung nach einem der vorhergehenden Ansprüche, **dadurch gekennzeichnet, daß** die flüssige Fettphase 5 bis 99 % des Gesamtgewichts der Zusammensetzung und besser 20 bis 75 % ausmacht.

24. Zusammensetzung nach einem der vorhergehenden Ansprüche, **dadurch gekennzeichnet, daß** sie eine Zusammensetzung zur Pflege und/oder zur Behandlung und/oder zum Schminken von Keratinsubstanzen ist.

25. Zusammensetzung nach einem der vorhergehenden Ansprüche, **dadurch gekennzeichnet, daß** sie ferner mindestens einen kosmetischen oder dermatologischen Wirkstoff enthält.

26. Zusammensetzung nach einem der vorhergehenden Ansprüche, **dadurch gekennzeichnet, daß** sie mindestens einen Zusatzstoff enthält, der unter Wasser, Antioxidantien, etherischen Ölen, Konservierungsmitteln, Neutralisationsmitteln, fettlöslichen Polymeren, Füllstoffen, Parfums und deren Gemischen ausgewählt ist.

27. Zusammensetzung nach einem der vorhergehenden Ansprüche, **dadurch gekennzeichnet, daß** sie als transparentes, wasserfreies, starres Gel und insbesondere als transparenter, wasserfreier Stift vorliegt.

28. Zusammensetzung nach einem der vorhergehenden Ansprüche, **dadurch gekennzeichnet, daß** das Farbmittel unter den lipophilen Farbmitteln, hydrophilen Farbmitteln, Pigmenten, Perlglanzpigmenten und deren Gemischen ausgewählt ist.

29. Zusammensetzung nach einem der vorhergehenden Ansprüche, **dadurch gekennzeichnet, daß** das Farbmittel in Mengenanteilen von 0,01 bis 40 % des Gesamtgewichts der Zusammensetzung und vorzugsweise 5 bis 25 % vorliegt.

30. Strukturierte Zusammensetzung zum Schminken der Haut, der Lippen und/oder der Hautanhangsgebilde, die mindestens ein Pigment in einer zum Schminken der Haut, der Lippen und/oder der Hautanhangsgebilde ausreichenden Menge und eine kontinuierliche flüssige Fettphase enthält, die mit mindestens einem Polymer mit einem Gewichtsmittel des Molekulargewichts von 1 000 bis 30 000 strukturiert ist, das a) ein Polymergerüst mit wiederkehrenden Kohlenwasserstoffeinheiten aufweist, die mindestens ein Heteroatom enthalten, und b) mindestens eine Fettkette als Seitenkette und/oder mindestens eine endständige Fettkette mit 12 bis 120 Kohlenstoffatomen aufweist, die an diese Einheiten gebunden sind, wobei die Fettketten 40 bis 98 % der Gesamtzahl der Einheiten mit Heteroatom und der Fettketten ausmachen, wobei die Zusammensetzung in Form eines Feststoffs vorliegt, wobei das Farbmittel, die flüssige Fettphase und das Polymer ein physiologisch akzeptables Medium bilden und wobei die Zusammensetzung ferner eine bei Raumtemperatur flüssige, amphiphile Verbindung enthält, die einen HLB-Wert unter 12 aufweist.

31. Zusammensetzung nach Anspruch 30, **dadurch gekennzeichnet, daß** die Zusammensetzung selbsttragend ist.

32. Zusammensetzung nach Anspruch 30, **dadurch gekennzeichnet, daß** sie als Feststoff mit einer Härte im Bereich von 0,19 bis 19,6 N (20 bis 2000 gf) und vorzugsweise 0,19 bis 8,82 N (20 bis 900 gf) vorliegt.

33. Zusammensetzung nach Anspruch 32, **dadurch gekennzeichnet, daß** das Polymer ein Polyamid ist.

34. Zusammensetzung nach einem der Ansprüche 32 bis 33, **dadurch gekennzeichnet, daß** die endständigen Fettketten über eine Estergruppe an das Kohlenstoffgerüst gebunden sind.

35. Zusammensetzung nach einem der Ansprüche 1 bis 32, **dadurch gekennzeichnet, daß** sie als Mascara in Stückform, Eyeliner, Make-up, Lippenstift, Rouge, Deodorant, Produkt zum Abschminken, Produkt zum Schminken des Körpers, Lidschatten, Wangenrouge oder Produkt gegen Augenringe vorliegt.

36. Zusammensetzung nach einem der vorhergehenden Ansprüche, **dadurch gekennzeichnet, daß** die Einheiten mit Heteroatom jeweils ein oder mehrere Heteroatome enthalten, die unter Stickstoff, Schwefel, Phosphor und deren Kombinationen gegebenenfalls in Kombination mit einem oder mehreren Sauerstoffatomen ausgewählt sind.

37. Zusammensetzung nach einem der vorhergehenden Ansprüche, **dadurch gekennzeichnet, daß** das strukturierende Polymer der Zusammensetzung eine Erweichungstemperatur über 70 °C aufweist, die bis zu 190 °C erreichen kann, und vorzugsweise eine Erweichungstemperatur im Bereich von 80 bis 130 °C und besser 80 bis 105 °C hat.

38. Zusammensetzung nach Anspruch 30 oder 31, wobei die Zusammensetzung beispielsweise in Form eines selbsttragenden oder als Lippenstift vorliegt.

39. Zusammensetzung nach Anspruch 30 oder 31 in Stiftform zum Schminken der Haut, der Lippen und/oder der Hautanhangsgebilde und insbesondere zum Schminken der Lippen.

40. Kosmetisches Verfahren zur Pflege, zum Schminken oder zur Behandlung von menschlichen Keratinsubstanzen, das umfaßt, eine kosmetische Zusammensetzung nach einem der vorhergehenden Ansprüche auf die Keratinsubstanzen aufzutragen.

41. Verwendung einer wirksamen Menge mindestens eines Polymers mit einem Gewichtsmittel des Molekulargewichts von 1 000 bis 30 000, das a) ein Polymergerüst mit wiederkehrenden Kohlenwasserstoffeinheiten, die mindestens ein Heteroatom enthalten, und b) mindestens eine Fettkette als Seitenkette und/oder mindestens eine endständige Fettkette mit 12 bis 120 Kohlenstoffatomen aufweist, die gegebenenfalls furiktionalisiert sind und die an diese Einheiten gebunden sind, wobei die Fettketten 40 bis 98 % der Gesamtzahl der Einheiten mit Heteroatom und der Fettketten ausmachen, in einer nicht therapeutischen kosmetischen Zusammensetzung oder zur Herstellung einer physiologisch akzeptablen Zusammensetzung, die eine kontinuierliche flüssige Fettphase und ein Farbmittel enthält, zur Strukturierung der Zusammensetzung in Form eines selbsttragenden Feststoffes mit einer Härte im Bereich von 0,19 bis 19,6 N (20 bis 2000 gf), wobei die Zusammensetzung ferner eine bei Raumtemperatur flüssige, amphiphile Verbindung mit einem HLB-Wert unter 12 aufweist und wobei die Zusammensetzung kein Wachs enthält.

42. Verwendung nach Anspruch 41 in einer nicht therapeutischen kosmetischen Zusammensetzung oder zur Herstellung einer physiologisch akzeptablen Zusammensetzung zur Strukturierung der Zusammensetzung in Form eines selbsttragenden Feststoffes mit einer Härte im Bereich von 0,19 bis 8,82 N (20 bis 900 gf) und besser 0,19 bis 5,88 N (20 bis 600 gf).

43. Verwendung nach Anspruch 41 oder 42, **dadurch gekennzeichnet, daß** das Polymer ein Polyamid ist, das endständige Gruppen mit Esterfunktion aufweist, die eine Kohlenwasserstoffkette mit 10 bis 42 Kohlenstoffatomen trägt.

44. Verwendung nach einem der Ansprüche 41 bis 43, **dadurch gekennzeichnet, daß** das Polymer mit einer amphiphilen Verbindung mit einem HLB-Wert im Bereich von 1 bis 7 und vorzugsweise 1 bis 5 kombiniert wird.

45. Verwendung einer kontinuierlichen flüssigen Fettphase, die im wesentlichen mit einer wirksamen Menge mindestens eines Polymers mit einem Gewichtsmittel des Molekulargewichts von 1 000 bis 30 000 strukturiert ist, das a) ein Polymergerüst mit wiederkehrenden Kohlenwasserstoffeinheiten aufweist, die mindestens ein Heteroatom enthalten, und b) mindestens eine Fettkette als Seitenkette und/oder mindestens eine endständige Fettkette mit 12 bis 120 Kohlenstoffatomen aufweist, die gegebenenfalls funktionalisiert sind und die an diese Einheiten gebunden sind, wobei die Fettketten 40 bis 98 % der Gesamtzahl der Einheiten mit Heteroatom und der Fettketten ausmachen, in einer nicht therapeutischen kosmetischen Zusammensetzung oder zur Herstellung einer physiologisch akzeptablen Zusammensetzung, die eine flüssige amphiphile Verbindung enthält, die einen HLB-Wert unter 12 und insbesondere im Bereich von 1 bis 7 und vorzugsweise 1 bis 5 aufweist, wobei die Zusammensetzung starr und insbesondere selbsttragend ist, kein Wachs enthält, glänzt und/oder nicht migriert.

46. Verwendung nach dem vorhergehenden Anspruch, wobei das Polymer ein Polyamid ist, das endständige Gruppen mit Esterfunktion aufweist, die eine Kohlenwasserstoffkette mit 10 bis 42 Kohlenstoffatomen trägt.

47. Verwendung einer kontinuierlichen flüssigen Fettphase, die im wesentlichen mit einer wirksamen Menge mindestens eines Polymers mit einem Gewichtsmittel des Molekulargewichts von 1 000 bis 30 000 strukturiert ist, das a) ein Polymergerüst mit wiederkehrenden Kohlenwasserstoffeinheiten aufweist, die mindestens ein Heteroatom enthalten, und b) mindestens eine Fettkette als Seitenkette und/oder mindestens eine endständige Fettkette mit 12 bis 120 Kohlenstoffatomen aufweist, die gegebenenfalls funktionalisiert sind und die an diese Einheiten gebunden sind, wobei die Fettketten 40 bis 98 % der Gesamtzahl der Einheiten mit Heteroatom und der Fettketten ausmachen, in einer nicht therapeutischen kosmetischen Zusammensetzung oder zur Herstellung einer physiologisch akzeptablen Zusammensetzung als Mittel, um die Migration der Zusammensetzung einzuschränken.

48. Verwendung nach einem der Ansprüche 45 bis 47, wobei die Zusammensetzung eine Härte im Bereich von 0,19 bis 19,6 N (20 bis 2000 gf), vorzugsweise 0,19 bis 8,82 N (20 bis 900 gf) und besser 0,19 bis 5,88 N (20 bis 600 gf) aufweist.

49. Kosmetisches Verfahren, um die Migration einer kosmetischen Zusammensetzung, die eine flüssige Fettphase enthält, einzuschränken, das darin besteht, die flüssige Fettphase mit einer wirksamen Menge mindestens eines Polymers mit einem Gewichtsmittel des Molekulargewichts von 1 000 bis 30 000 zu strukturieren, das a) ein Polymergerüst mit wiederkehrenden Kohlenwasserstoffeinheiten aufweist, die mindestens ein Heteroatom enthalten, und b) mindestens eine Fettkette als Seitenkette und/oder mindestens eine endständige Fettkette mit 12 bis 120 Kohlenstoffatomen aufweist, die gegebenenfalls funktionalisiert sind und die an diese Einheiten gebunden sind, wobei die Fettketten 40 bis 98 % der Gesamtzahl der Einheiten mit Heteroatom und der Fettketten ausmachen.
